# FASCICULE DE BREVET EUROPEEN

(11) **EP 0 954 290 B1**
(45) Date de publication et mention de la délivrance du brevet: **21.05.2003**
(21) Numéro de dépôt: 97915220.4
(22) Date de dépôt: 04.04.1997
(51) Int. Cl.: A61K 9/20

(54) **COMPRIME PHARMACEUTIQUE A LIBERATION CONTROLEE CONTENANT UN SUPPORT A BASE D'AMYLOSE RETICULE ET D'HYDROXYPROPYLMETHYLCELLULOSE**
PHARMAZEUTISCHE TABLETTE MIT VERZÖGERTER FREISETZUNG, ENTHALTEND EINE MATRIX AUS QUERVERNETZTER AMYLOSE UND HYDROXYPROPYL METHYLZELLULOSE
CONTROLLED-RELEASE PHARMACEUTICAL TABLET CONTAINING A CARRIER BASED ON CROSS-LINKED AMYLOSE AND HYDROXYPROPYLMETHYLCELLULOSE

(30) Priorité: 10.04.1996 CA 2173818
(43) Date de publication de la demande: 10.11.1999
(73) Titulaire: Labopharm Inc., Laval, Québec H7V 3Z3 (CA)
(72) Inventeur: CHOUINARD, François, Laval, Québec H7L 4X7 (CA); JACQUES, Wilfrid, Longueuil, Québec J4K 3E8 (CA)
(74) Mandataire: Guerre, Dominique
(86) Numéro de dépôt international: CA9700229
(87) Numéro de publication internationale: WO97037639

(56) Documents cités:
- WO-A-94/02121
- WO-A-94/21236
- US-A- 5 456 921

## Description

### DOMAINE TECHNIQUE DE L'INVENTION

La présente invention a pour objet un perfectionnement à l'invention décrite et revendiquée dans le brevet canadien n° 2.041.774 délivré le 19 avril 1994 et son équivalent américain n° 5.456.921 délivré le 10 octobre 1995, tous deux au nom de la Demanderesse.

Plus précisément, l'invention a pour objet un comprimé pharmaceutique à libération contrôlée contenant un principe actif en association avec un support à base d'amylose réticulé dans lequel de l'hydroxypropylméthylcellulose (HPMC) ayant une viscosité supérieure ou égale à 4000 cps (mPas) est ajoutée comme adjuvant.

L'invention a également pour objet l'usage d'HPMC ayant une viscosité supérieure ou égale à 4000 cps (mPas) comme adjuvant dans un comprimé du type ci-dessus défini, dans le but de contrôler l'effet des enzymes et notamment de l'α-amylase, présents en milieu intestinal sur l'amylose réticulé et de là réduire la dépendance de la vitesse de libération face à la concentration d'enzymes présents dans le milieu.

### BRÈVE DESCRIPTION DE L'ART ANTÉRIEUR

Les brevets canadien n° 2.041.774 et américain n° 5.456.921 ci-dessus mentionnés décrivent tous deux des comprimés pharmaceutiques destinés à être administrés oralement en vue de délivrer ou "libérer" une dose de médicament de façon contrôlée sur une période de temps donné. Les comprimés en question sont décrits comme contenant jusqu'à 60% en poids d'au moins un principe actif qui peut être de n'importe quelle nature. Les comprimés comprennent également au moins 40% en poids d'un support ou "véhicule" constitué d'amylose réticulé à l'aide d'un agent de réticulation dans des quantités correspondant à 0,1 à 10 grammes d'agent de réticulation pour 100 grammes d'amylose, la quantité d'agent de réticulation préférée étant de 0,5 à 7,5 grammes et plus préférablement encore de 1 à 6 grammes pour 100 grammes d'amylose. Cet amylose réticulé, ci-après appelé *Contramid®,* est préférablement obtenu en utilisant de l'épichlorhydrine ou du 2,3-dibromopropanol comme agent de réticulation, puisque l'amylose réticulé par ces deux produits est approuvé depuis de nombreuses années par la plupart des organismes de contrôle des médicaments, dont notamment la *Food & Drug Administration* américaine.

Le principal avantage du *Contramid®* est qu'il assure une libération contrôlée à vitesse constante (ordre O), contrairement à la plupart des véhicules utilisés actuellement dans les comprimés à libération contrôlée, où le médicament est libéré par diffusion, selon une cinétique fickienne (la fraction libérée cumulative est proportionnelle à la racine carrée du temps).

D'un point de vue pratique, le *Contramid*® forme en milieu aqueux un hydrogel poreux qui sert de support au médicament et assure une libération contrôlée de celui-ci lorsqu'il est administré par voie orale. En milieu intestinal, cet hydrogel est sensible à l'action d'enzymes digestifs qui attaquent les chaînes d'amylose et dégradent le comprimé, ce qui assure sa désintégration dans le tube digestif. L'enzyme α-amylase est, à ce titre, particulièrement efficace pour accélérer la libération dans les comprimés et son utilisation comme adjuvant lors de la fabrication des comprimés a d'ailleurs fait l'objet d'une demande de brevet internationale qui a été publiée le 3 février 1994 sous le n° WO 94/02121, au nom de la Demanderesse.

Les comprimés ainsi produits sont efficaces et assurent une bonne libération contrôlée dans la plupart des patients. Toutefois, il existe une certaine disparité d'un patient à l'autre, compte tenu du fait qu'il existe des variations importantes de l'activité enzymatique selon les individus et l'apport en nourriture. Puisque le *Contramid®* est sensible à l'amylase pancréatique, ces variations peuvent devenir des inconvénients à la commercialisation des comprimés, du moins avec certains principes actifs.

L'ajout d'un adjuvant permettant de contrôler l'effet des enzymes serait donc un atout important pour qui désirerait exploiter sans aucune restriction, à l'échelle industrielle, les propriétés uniques du *Contramid®*.

### RÉSUMÉ DE L'INVENTION

La présente invention résulte d'une découverte très surprenante faite par les inventeurs, à savoir que l'ajout au *Contramid®* d'un adjuvant extrêmement spécifique, dans des quantités également très spécifiques, permet de protéger les comprimés pharmaceutiques obtenus contre toute variation majeure de la vitesse de relâchement du médicament due à la dégradation plus ou moins rapide du *Contramid*® par les enzymes présents en milieu intestinal.

L'adjuvant en question est un hydrogel bien connu et couramment utilisé dans le domaine pharmaceutique, à savoir l'hydroxypropylméthylcellulose (HPMC). Toutefois, cet adjuvant ne s'avère efficace que si sa viscosité est supérieure ou égale à 4000 centipoises (cps) (mPa) et si la quantité ajoutée est comprise entre 10 et 30% en poids par rapport au poids total du comprimé. En dessous de 10%, la quantité d'HPMC ajoutée est insuffisante pour obtenir le résultat escompté. Au dessus de 30%, la quantité de HPMC ajoutée devient trop grande et affecte la nature même du support, qui n'est plus constitué principalement de *Contramid®*, ce qui entraîne une libération qui tend vers la cinétique fickienne.

Dans le cadre de l'invention, il a donc été découvert que l'ajout d'HPMC d'une viscosité supérieure ou égale à 4000 cps (mPa) au *Contramid*® procure à ce dernier des propriétés uniques, imprévisibles et qui ne pouvaient être démontrées au niveau actuel des connaissances sur ce produit, à savoir une plus grande résistance au milieu enzymatique et donc une dépendance moins prononcée face à la concentration en enzyme dans le milieu.

Cette découverte est surprenante dans la mesure où des essais similaires effectués en ajoutant d'autres types de polymères utilisés dans le domaine pharmaceutique et considérés comme des substituts possibles à l'HPMC, tels que l'éthylcellulose, le méthylcellulose, l'hydroxypropylcellulose (HPC) ou le Carbomer, se sont avérés négatifs, comme il sera démontré plus loin.

L'invention telle que ci-après revendiquée a donc pour premier objet un comprimé pharmaceutique destiné à l'administration orale d'une quantité donnée d'au moins un principe actif en vue d'obtenir une libération contrôlée de celui-ci, ledit composé contenant jusqu'à 60% en poids du principe actif mélangé et compressé à au moins 40% en poids d'un support à base d'amylose réticulé à l'aide d'un agent de réticulation dans des quantités correspondant à 0,1 à 10 grammes et de préférence 1 à 6 grammes d'agent de réticulation pour 100 grammes d'amylose. Ce comprimé est caractérisé en ce que son support contient:
de 30 à 90% d'amylose réticulé; et
de 10 à 30% d'hydroxypropylméthycelluylose (HPMC) ayant une viscosité supérieure ou égale à 4000 cps (mPa),
les pourcentages en question étant exprimés en poids par rapport au poids total du comprimé.

L'invention a également pour second objet l'usage d'hydroxylpropylméthylcellulose (HPMC) ayant une viscosité supérieure ou égale à 4000 cps comme adjuvant pour contrôler l'effet des enzymes sur un support à base d'amylose réticulé à l'aide d'un agent de réticulation dans des quantités correspondant à 0,1 à 10 grammes d'agent de réticulation pour 100 gammes d'amylose, dans un comprimé pharmaceutique pour administration orale avec libération contrôlée contenant jusqu'à 60% en poids de principe actif.

Les comprimés ainsi obtenus montrent une excellente résistance au milieu enzymatique et donc une dépendance moins prononcée à la concentration d'enzymes dans le milieu. Ils montrent aussi une meilleure résistance mécanique en usage, ce qui est un atout du point de vue commercial.

L'invention et ses divers avantages seront mieux appréciés à la lecture de la description générale non limitative qui va suivre, incluant notamment les résultats d'essais effectués par les inventeurs.

### DESCRIPTION GÉNÉRALE DE L'INVENTION

Tel que précédemment indiqué, l'invention concerne donc la préparation de comprimés pharmaceutiques destinés à l'administration orale d'un principe actif en vue d'obtenir une libération contrôlée de ce dernier sur une période de temps donné. Les composés en question contiennent jusqu'à 60% en poids du principe actif mélangé et compressé à au moins 40% en poids d'un support à base d'amylose réticulé à l'aide d'un agent de réticulation dans des quantités correspondant à 0,1 à 10 grammes d'agent de réticulation pour 100 grammes d'amylose.

Par libération contrôlée, on entend une libération à vitesse presque constante (linéaire) pour une période pouvant aller jusqu'à plus de 20 heures.

Le principe actif utilisé peut être de n'importe quel type administrable oralement. À titre d'exemples non limitatifs, il peut être choisi parmi les sédatifs, les analgésiques, les médicaments antiacides ou les anti-inflammatoires, les vasodilatateurs coronaires ou cérébraux, les stimulants, les antihistaminiques, les décongestionnants, les vasoconstricteurs, les anticoagulants, les médicaments anti-arythmiques ou anti-hypertensifs, les médicaments anaboliques ou hyper- ou hypoglycémiques, les diurétiques, les agents antiasthmatiques, antipyrétiques ou antiémétiques, les agents antispasmodiques, etc...

L'amylose réticulé ou *Contramid*® utilisé est du type décrit en détail dans le brevet canadien 2.041.774 ci-dessus mentionné. Ce produit est de préférence préparé avec de 1 à 6 grammes d'agent de réticulation pour 100 grammes d'amylose. L'agent de réticulation est de préférence l'épichlorhydrine. Ce pourrait toutefois être n'importe quel autre agent de réticulation pharmaceutiquement acceptable, tel le 2,3-dibromopropanol. Le *Contramid*® se présente sous la forme de particules. De préférence, au moins 50% de celles-ci ont une taille comprise entre 25 et 700 microns (µm).

Le principe actif, qui est de préférence également sous la forme d'une poudre, est mélangée au support à base de *Contramid*® et le mélange ainsi obtenu est compressé pour obtenir les comprimés désirés. La compression s'effectue de préférence à une pression d'au moins 0,15 tonne par cm².

L'invention réside essentiellement dans le fait que le support à base d'amylose réticulé utilisé pour la préparation des comprimés contient de 30 à 90% de *Contramid®* et de 10 à 30% d'hydroxypropylméthylcellulose (HPMC) ayant une viscosité supérieure ou égale à 4000 cps (mPas), les pourcentages en question étant exprimés en poids par rapport au poids total du comprimé.

L'HPMC utilisée est de préférence choisi parmi les HPMC identifiées par les chiffres 2208 et 2910 dans le Recueil de pharmacopée américain USP, 23° édition (USP Standards, grade XXIII). La viscosité du HPMD est déterminée en fonction de ces standards dans une solution aqueuse à 2% à une température de 20 ± 0,1 °C.

L'HPMC 2208 a une teneur en méthoxyle comprise entre 19 et 24%et une teneur en hydroxypropoxyle comprise entre 4 et 12%. Å titre d'exemple, ce produit est commercialisé sous des viscosités de 100, 4000, 15000 et 100000 par THE DOW CHEMICAL CO sous les marques *METHOCEL®* K- 100, 4M, 15M et 100M.

L'HPMC 2910 a une teneur en méthoxyle comprise entre 28 et 30% et une teneur en hydroxypropoxyle comprise entre 7 et 12%. À titre d'exemple, ce produit est commercialisé sous des viscosités de 4000 et 100000 par THE DOW CHEMICAL CO sous les marques *METHOCEL* E-4M et 100M.

Le support à base d'amylose réticulé peut aussi contenir un ou plusieurs autres ingrédients usuels couramment utilisés dans la préparation de comprimés pharmaceutiques, tels que:
- des agents de remplissage (fillers) comme du lactose ou du sucrose, en quantité pouvant aller jusqu'à 40% en poids;
- des agents d'écoulement comme du dioxyde de silicium, en quantité pouvant aller jusqu'à 10% en poids;
- des agents de granulation (binders) en quantité pouvant aller jusqu'à 10% en poids;
- des agents de démoulage comme du stéarate de magnésium en quantité pouvant aller jusqu'à 5% en poids; et/ou
- des désintégrants en quantité pouvant aller jusqu'à 5% en poids.

Le comprimé peut être de type matriciel ou à double noyau.

Dans le premier cas, l'HPMC utilisée est de préférence choisie parmi les HPMC 2208 et 2910 dont la viscosité est supérieure à 4000. De préférence, on utilisera l'HPMC 2208 ayant une viscosité de 100000 cps.

Dans le cas où le comprimé est à double noyau, l'HPMC utilisée est de préférence choisi aussi parmi les HPMC 2208 et 2910 dont la viscosité est non seulement supérieure mais égale à 4000 cps (mPas). Le comprimé en question inclut un coeur contenant une certaine quantité de principe actif et un recouvrement externe contenant une autre quantité du même ou d'un autre principe actif mélangée et compressée avec le support contenant de l'amylose réticulé et de l'HPMC. Le noyau peut aussi inclure un support à base d'amylose réticulé et inclure de l'HPMC.

Les comprimés à double noyau sont particulièrement utiles dans certains cas dans la mesure où leur recouvrement permet d'augmenter la flexibilité de libération, qui peut être lente au début et rapide à la fin ou vice versa, et d'augmenter le dosage, surtout lorsque le principe actif que l'on cherche à administrer est très soluble dans l'eau. Dans la plupart des cas, ils assurent une libération en deux étapes.

Tel que précédemment indiqué, l'HPMC, tout comme d'autres polymères tel que l'hydroxypropylcellulose (HPC) ou le Carbomer (tel que celui commercialisé par B.F. GOODRICH sous la marque *CARBOPOL®*), sont déjà utilisés pour la fabrication de comprimés en vue d'obtenir la libération contrôlée d'un médicament. À ce sujet, on peut se référer à titre d'exemple non limitatif au brevet américain no 3.065.143 de 1962 ou encore au brevet canadien n° 1.188.614 de 1985.

Le brevet américain no 3,065.143 enseigne que l'HPMC peut être utilisée pour la préparation de comprimés à libération contrôlée, à condition d'être utilisée en quantité supérieure à 30% en poids. Ce brevet décrit que l'HPMC forme une barrière mucilagineuse, composée de gomme gonflée sous l'action de l'eau, dont l'érosion progressive dans le tractus gastro-intestinal assure la libération contrôlée voulue (voir exemple 1 qui donne les temps de désintégration in vitro). Ce brevet fait état d'une désintégration lente du comprimé avec libération subséquente du principe actif sur une période de plus de 4 heures. Ceci est différent de la présente invention où le comprimé gonfle à peine et ne se désagrège pas du tout pendant plus de 20 heures in vitro en milieu enzymatique. En outre, les temps de libération dans le cas des comprimés selon l'invention sont beaucoup plus long.

Le brevet canadien no 1,188.614 enseigne quant à lui que l'HPMC peut être utilisée avec des excipients mineurs, pour faire des comprimés contenant de 70 à 95% de principe actif, tout en obtenant une libération lente in vitro. Ce système, comme celui mentionné dans le brevet américain n° 3.065.143, forme une "soft mucilaginous gel barrier" qui libère le médicament par diffusion, selon une cinétique fickienne (la fraction libérée cumulative est proportionnelle à la racine carrée du temps).

Le brevet ne suggère pas d'utiliser l'HPMC pour donner à un comprimé d'amylose réticulé une résistance face à l'action de l'α-amylase présente dans les liquides intestinaux.

De façon différente, le comprimé d'amylose réticulé contenant de l'HPMC selon l'invention reste sous sa forme d'origine lorsqu'il est placé en milieu aqueux, et ce pour au moins 24 heures (le temps nécessaire à libérer le principe actif). Aucun mucilage ne se forme et la libération a généralement une vitesse constante (ordre 0) et dure plus longtemps que dans le cas des exemples du brevet canadien n° 1.188.614.

Il ne s'agit donc pas d'une même technologie.

Afin de démontrer l'exactitude des affirmations et informations qui précèdent, des essais ont été effectués.

### Fabrication des comprimés

Pour ces essais, des comprimés pharmaceutiques de type matriciel et à double noyau incluant du *Contramid*® comme support avec ou sans ajout de HPMC ont été préparés en utilisant la méthodologie décrite en détail dans le brevet canadien n° 2.041.774. D'autres polymères gélifiants ont aussi été testés pour fins de comparaison.

Les comprimés de type matriciel préparés avaient tous un poids de 500 mg et contenaient 50 mg d'acide acétylsalicylique (ASA) comme médicament type. De l'HPMC sous une concentration de O et 30% en poids et du *Contramid®* sous une concentration de 60-90% en poids, complétaient la formule avec 0,25% en poids de stéarate de magnésium comme agent de démoulage. Les comprimés en question étaient de type cylindrique biconvexe avec un diamètre de 12,7 mm.

Le *Contramid*® utilisé pour leur fabrication a été préparé en utilisant 3,5 grammes d'épichlorhydrine comme agent de réticulation pour 100 grammes d'amylose, en suivant là encore la méthodologie décrite en détail dans le brevet canadien n° 2.041.774.

Les comprimés de type double noyau préparés avaient un coeur contenant 128 mg de chlorhydrate de pseudoéphédrine comme médicament type, mélangés à 44 mg de *Contramid®* utilisé comme support, le tout étant enveloppé d'un recouvrement contenant 72 mg de chlorhydrate de pseudoéphédrine, 406 mg de *Contramid*® et 120 mg (20%) de HPMC 2208/100000. Le *Contramid*® utilisé pour leur fabrication a été préparé en utilisant 2 grammes d'épichlorhydrine comme agent de réticulation, pour 100 grammes d'amylose.

L'HPMC se retrouvant sous plusieurs types de viscosité variable, les dénominations ont été abrégées de la façon suivante:
- HPMC 2208 de 100 cps (mPas) =: HPMC 2208/100
- HPMC 2208 de 4000 cps (mPas) =: HPMC 2208/4000
- HPMC 2208 de 100000 cps (mPas) =: HPMC 2208/100000
- HPMC 2910 de 4000 cps (mPas) =: HPMC 2910/4000

### Dosage in vitro

Les comprimés ainsi préparés ont été testés par dissolution en milieu aqueux sous agitation mécanique à 37°C. Tous les essais sont faits au moins en duplicata dans les conditions de dissolution suivantes:

| | |
|---|---|
| Appareil utilisé | U.S.P. Dissolution apparatus type 3 |
| Agitation | 10 trempages par minute |
| Milieu de dissolution | 2 heures en milieu acide pH 1,2; |
| | 12 heures en milieu phosphate pH 7,0 avec ou sans enzyme (la |
| | concentration en enzyme α-amylase bactérienne (bacillus) variant de 0 à 72 U.I./ml selon essai); et |
| | 10 heures en milieu phosphate pH 7,0. |

### Brève description des dessins

Les résultats obtenus au cours des essais ainsi effectués sont illustrés sur les dessins annexés dans lesquels:
la figure 1 est une courbe illustrant le profil de dissolution de comprimés de 500 mg contenant 50 mg d'ASA, du *Contramid®* et différentes concentrations en HPMC 2208/100000 avec 18 U.I./ml d'enzyme dans le milieu phosphate;
la figure 2 est une courbe illustrant le profil de dissolution de comprimés de 500 mg contenant 50 mg d'ASA, du *Contramid®* et 20% de HPMC 2208/100000 avec différentes concentrations d'enzyme dans le milieu phosphate;
la figure 3 est une courbe illustrant le profil de dissolution de comprimés de 500 mg contenant 50 mg d'ASA et du *Contramid*® sans HPMC avec différentes concentrations d'enzyme dans le milieu phosphate;
la figure 4 est une courbe illustrant le profil de dissolution de comprimés de 500 mg contenant 50 mg d'ASA, du *Contramid*® et 20% de HPMC 2208/100 avec différentes concentrations d'enzyme dans le milieu phosphate;
la figure 5 est une courbe illustrant le profil de dissolution de comprimés de 500 mg contenant 50 mg d'ASA, du *Contramid®* et 20% de polymère gélifiant avec 18 U.l./mt d'enzyme dans le milieu phosphate (les essais avec le Carbomer ont été effectués à une concentration de 10%);
la figure 6 est une courbe illustrant le profil de dissolution de comprimés de chlorhydrate de pseudoéphédrine à double noyau de *Contramid®* contenant 20% de HPMC 2208/1000000 avec différentes concentrations d'enzyme dans le milieu phosphate; et
la figure 7 est une courbe illustrant le profil de dissolution de comprimés de chlorhydrate de pseudoéphédrine à double noyau de *Contramid*® contenant 20% de différents types de HPMC avec 18 U.I./ml d'enzyme dans le milieu phosphate.

### Comprimés de type matriciel

### (a) Effet de la concentration en HPMC

En milieu enzymatique de 18 U.I./ml, la concentration en HPMC a eu un effet direct sur la résistance du comprimé à l'enzyme et sur le profil de libération du comprimé. Alors qu'à faible concentration (<10%), le comprimé avait un profil découpé et peu reproductible, au-delà de 10%, la courbe est plus linéaire. La libération se prolonge considérablement à 20 et 30%.

Ceci montre que la matrice change radicalement de propriété à mesure que la concentration en HPMC augmente. Avec un minimum de 10% en HPMC, le comprimé a une meilleure résistance enzymatique ce qui donne des profils plus linéaires et reproductibles. À plus de 20%, le comprimé a une très bonne résistance enzymatique.

### (b) Protection contre le milieu enzymatique

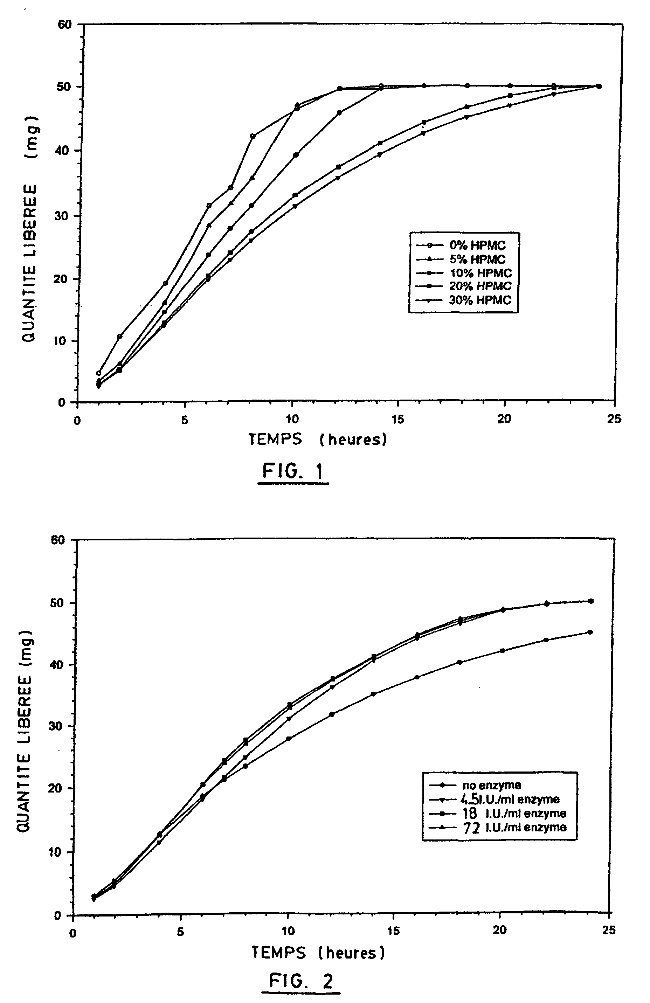

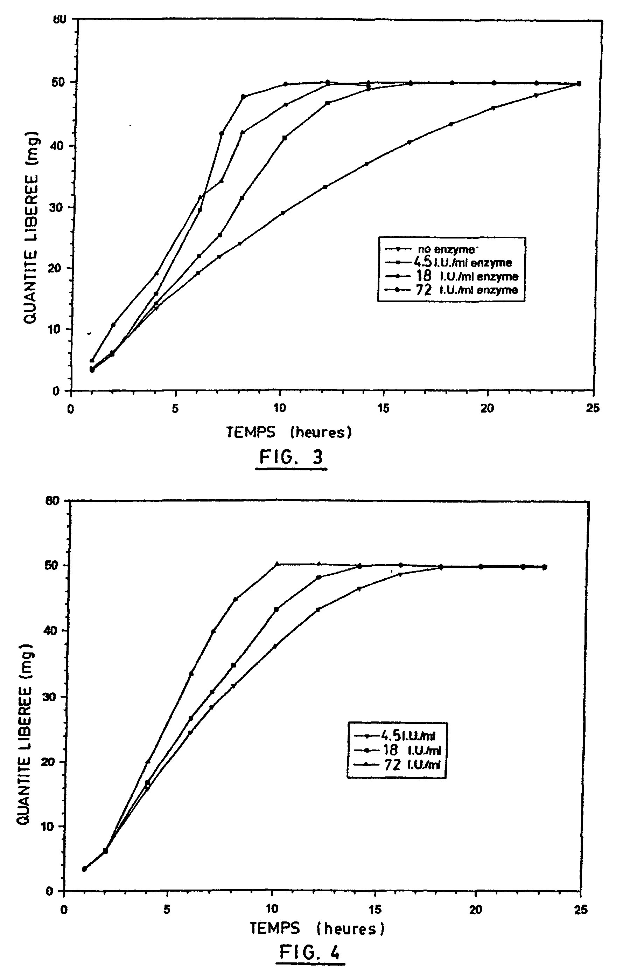

Les figures 2 et 3 montrent que le HPMC 2208/100000 a un effet direct sur la résistance enzymatique du comprimé. Avec 20% d'HPMC 2208/100000 ajouté dans le support de *Contramid®,* l'influence de la concentration en enzyme sur le profil est minime avec, cependant, une inflexion marquée vers le bas en milieu sans enzyme. Un comprimé sans HPMC 2208/100000 devient cependant beaucoup plus sensible à l'enzyme, même à faible concentration, alors qu'en milieu non enzymatique, la libération est presque identique à un comprimé avec 20% d'HPMC 2208/100000. Cette dernière observation démontre que l'HPMC en soi n'agit pas directement sur les propriétés de libération du *Contramid*® mais plutôt sur la sensibilité du comprimé à l'enzyme.

À titre de comparaison, de l'HPMC 2208/100 a été testée dans des conditions similaires. La figure 4 montre que l'effet de l'enzyme est beaucoup plus marqué lorsque l'HPMC a une viscosité de 100, si on la compare à l'HPMC ayant une viscosité de 100000. La masse moléculaire de l'HPMC aurait donc un effet critique sur la résistance à la dégradation par l'enzyme.

### (c) Effet du type de polymère

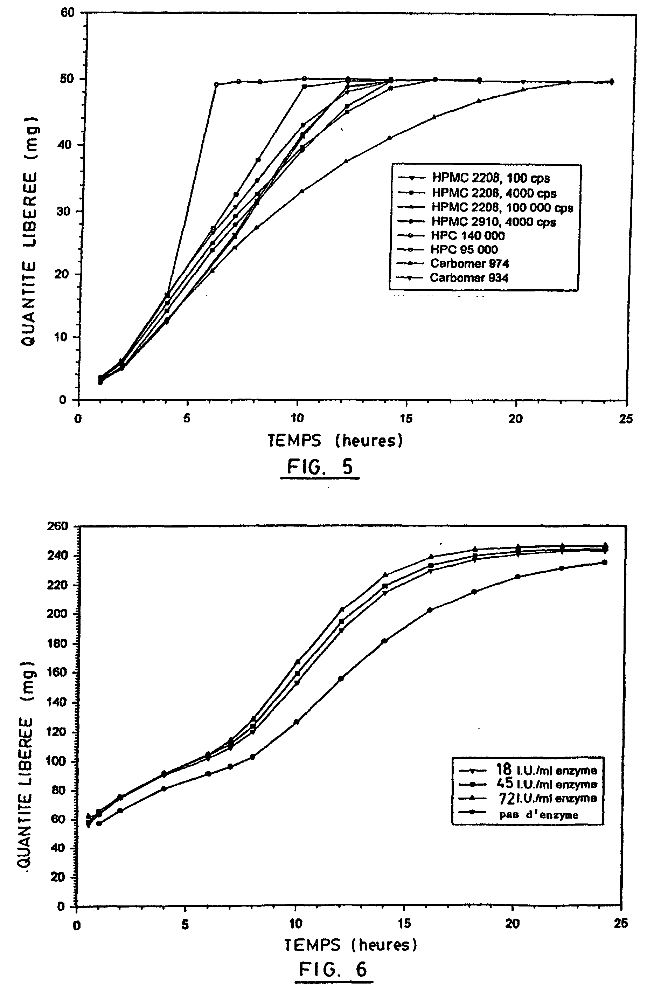

Des essais effectués sur plusieurs polymères capables de former un hydrogel en milieu aqueux ont révélé la particularité du l'HPMC 2208/100000. La figure 5 montre que les autres polymères, dont même les HPMC de viscosité égale à juste 4000 cps (mPas) entraînant tous une libération rapide du principe actif. On note toutefois que l'effet protecteur est optimal avec de l'HPMC de haute viscosité.

### Comprimés à double noyau

Les résultats ci-dessous analysés sont ceux qui ont été obtenus avec les comprimés à double noyau.

La figure 6 montre que les comprimés à double noyau résistent bien au milieu enzymatique avec du HPMC 2208/100000.

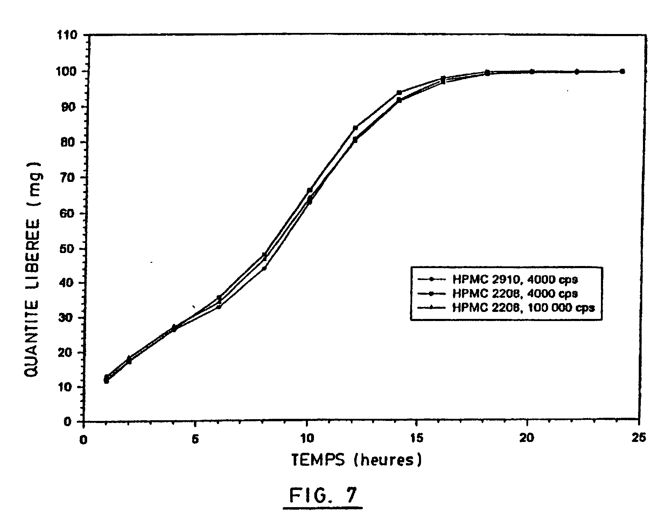

La figure 7 montre aussi que cet effet de protection est aussi prononcé même lorsque l'HPMC utilisée est de viscosité aussi basse que 4000 cps. (mPas).

Il va de soi que diverses modifications pourraient être apportées à ce qui vient d'être décrit de façon générale sans pour autant sortir du cadre de l'invention telle que définie dans les revendications annexées.

## Revendications

1. Comprimé pharmaceutique pour administration orale avec libération contrôlée d'une quantité donnée d'au moins un principe actif, ledit comprimé contenant jusqu'à 60 % en poids dudit principe actif mélangé et compressé à au moins 40 % en poids d'un support à base d'amylose réticulé à l'aide d'un agent de réticulation dans des quantités correspondant à 0,1 à 10 grammes d'agent de réticulation pour 100 grammes d'amylose, **caractérisé en ce que** ledit support contient :
de 30 à 90 % d'amylose réticulé, et
de 10 à 30% d'hydroxypropylméthylcellulose (HPMC) ayant une viscosité supérieure ou égale à 4 000 mPa.s
lesdits pourcentages étant exprimés en poids par rapport au poids total du comprimé.

2. Comprimé selon la revendication 1, **caractérisé en ce que** le support contient également au moins un ingrédient supplémentaire choisi dans le groupe constitué par les agents de remplissage, les agents d'écoulement, les agents de granulation, les agents de démoulage et les désintégrants pharmaceutiquement acceptables.

3. Comprimé selon la revendication 2, **caractérisé en ce que** le principe actif et le support à base d'amylose réticulé se présentent sous la forme de poudres qui sont mélangées et compressées pour obtenir le comprimé désiré et **en ce que** l'amylose réticulé utilisé a été préparé avec de 1 à 6 grammes d'agent de réticulation pour 100 grammes d'amylose.

4. Comprimé selon l'une quelconque des revendications 1 à 3, **caractérisé en ce que** ledit comprimé est de type matriciel et **en ce que** l'HPMC présente dans le support a une viscosité supérieure à 4 000 mPa.s.

5. Comprimé selon la revendication 4, **caractérisé en ce que** l'HPMC présente dans le support est de type HPMC 2208.

6. Comprimé selon la revendication 5, **caractérisé en ce que** l'HPMC présente dans le support est de type HPMC 2208 et a une viscosité égale à 100 000 mPa.s

7. Comprimé selon la revendication 6, **caractérisé en ce qu'**il contient 20 % en poids d'HPMC 2208 de viscosité égale à 100 000 mPa.s.

8. Comprimé selon la revendication 7, **caractérisé en ce qu'**il contient 10 % en poids de principe actif et **en ce que** l'amylose réticulé utilisé a été préparé avec 3,5 grammes d'épichlorhydrine utilisée comme agent de réticulation pour 100 grammes d'amylose.

9. Comprimé selon la revendication 4, **caractérisé en ce que** l'HPMC présente dans le support est de type HPMC 2910.

10. Comprimé selon l'une quelconque des revendications 1 à 3, **caractérisé en ce que** ledit comprimé est de type à double noyau.

11. Comprimé selon la revendication 10, **caractérisé en ce que** ledit comprimé à double noyau inclut un coeur contenant une certaine quantité de principe actif et un recouvrement externe contenant une autre quantité du même ou d'un autre principe actif mélangée et compressée avec ledit support contenant de l'amylose réticulé et de l'HPMC.

12. Comprimé selon la revendication 11, **caractérisé en ce que** le noyau inclut également un support à base d'amylose réticulé.

13. Comprimé selon la revendication 11 ou 12, **caractérisé en ce que** l'HPMC présente dans le ou les supports est choisi dans le groupe constitué par celles de type HPMC 2208 et HPMC 2910.

14. Utilisation d'HPMC selon la revendication 1, ayant une viscosité supérieure ou égale à 4 000 mPa.s dans un médicament constitué par un comprimé pharmaceutique pour administration orale avec libération contrôlée contenant jusqu'à 60% en poids de principe actif.

15. Utilisation selon la revendication 14, en tant qu'adjuvant pour contrôler l'effet des enzymes sur un support à base d'amylose réticulé à l'aide d'un agent de réticulation dans des quantités correspondant à 0,1 grammes d'agent de réticulation pour 100 grammes d'amylose.

## Patentansprüche

1. Pharmazeutische Tablette zur oralen Verabreichung mit protrahierter Freigabe mindestens eines Wirkstoffs, wobei die Tablette bis zu 60 Gew.-% des Wirkstoffs in Abmischung und Verpressung mit mindestens 40 Gew.-% eines Trägers auf Basis von mit Hilfe eines Vernetzungsmittels in einer Menge von 0,1 bis 10 Gramm Vernetzungsmittel pro 100 Gramm Amylose vernetzter Amylose enthält, **dadurch gekennzeichnet, daß** der Träger
30 bis 90% vernetzte Amylose sowie
10 bis 30% Hydroxypropylmethylcellulose (HPMC)
mit einer Viskosität von 4 000 mPa.s oder darüber enthält, wobei sich die in Gewichtsprozent angegebenen Prozentsätze auf das Gesamtgewicht der Tablette beziehen.

2. Tablette nach Anspruch 1, **dadurch gekennzeichnet, daß** der Träger auch mindestens einen zusätzlichen Bestandteil aus der Gruppe pharmazeutisch unbedenklicher Füllstoffe, Fließregulierungsmittel, Granulierhilfsmittel, Formentrennmittel und Sprengmittel enthält.

3. Tablette nach Anspruch 2, **dadurch gekennzeichnet, daß** der Wirkstoff und der Träger auf Basis von vernetzter Amylose in Form von Pulvern vorliegen, die vermischt und komprimiert werden, wodurch man die gewünschte Tablette erhält, sowie dadurch, daß die verwendete vernetzte Amylose mit 1 bis 6 Gramm Vernetzungsmittel pro 100 Gramm Amylose hergestellt wurde.

4. Tablette nach einem der Ansprüche 1 bis 3, **dadurch gekennzeichnet, daß** die Tablette zum Typ der Matrixtabletten gehört und daß die in dem Träger vorhandene HPMC eine Viskosität von über 4 000 mPa.s aufweist.

5. Tablette nach Anspruch 4, **dadurch gekennzeichnet, daß** die in dem Träger vorliegende HPMC vom Typ HPMC 2208 ist.

6. Tablette nach Anspruch 5, **dadurch gekennzeichnet, daß** die in dem Träger vorliegende HPMC vom Typ HPMC 2208 ist und eine Viskosität von 100 000 mPa.s aufweist.

7. Tablette nach Anspruch 6, **dadurch gekennzeichnet, daß** sie 20 Gew.-% HPMC 2208 mit einer Viskosität von 100 000 mPa.s aufweist.

8. Tablette nach Anspruch 7, **dadurch gekennzeichnet, daß** sie 10 Gew.-% Wirkstoff aufweist und daß die verwendete vernetzte Amylose mit 3,5 Gramm Epichlorhydrin als Vernetzungsmittel pro 100 Gramm Amylose hergestellt wurde.

9. Tablette nach Anspruch 4, **dadurch gekennzeichnet, daß** die in dem Träger vorliegende HPMC vom Typ HPMC 2910 ist.

10. Tablette nach einem der Ansprüche 1 bis 3, **dadurch gekennzeichnet, daß** die Tablette vom Doppelkerntyp ist.

11. Tablette nach Anspruch 10, **dadurch gekennzeichnet, daß** diese Doppelkerntablette einen Tablettenkern mit einer bestimmten Wirkstoffmenge und eine äußere Hülle mit einer anderen Menge des gleichen oder eines anderen Wirkstoffs in Abmischung und Verpressung mit dem Träger, der vernetzte Amylose und HPMC enthält, beinhaltet.

12. Tablette nach Anspruch 11, **dadurch gekennzeichnet, daß** der Kern ebenfalls einen Träger auf Basis von vernetzter Amylose beinhaltet.

13. Tablette nach Anspruch 11 oder 12, **dadurch gekennzeichnet, daß** die in dem Träger bzw. den Trägern vorhandene HPMC aus den Typen HPMC 2208 und HPMC 2910 ausgewählt ist.

14. Verwendung von HPMC nach Anspruch 1, mit einer Viskosität von 4 000 mPa.s oder darüber in einem Arzneimittel, das aus einer pharmazeutischen Tablette zur oralen Verabreichung mit protrahierter Freigabe, die bis zu 60 Gew.-% Wirkstoff enthält, besteht.

15. Verwendung nach Anspruch 14, als Hilfsmittel zur Kontrolle der Wirkung von Enzymen auf einem Träger auf Basis von mit Hilfe eines Vernetzungsmittels in einer Menge von 0,1 Gramm Vernetzungsmittel pro 100 Gramm Amylose vernetzter Amylose.

## Claims

1. Controlled release pharmaceutical tablet for the oral administration of a given quantity of at least one active ingredient, said tablet comprising up to 60% by weight of said active ingredient mixed and compressed with at least 40% by weight of a carrier based on amylose cross-linked with the aid of a cross-linking agent in quantities corresponding to 0.1 to 10 grams of cross-linking agent per 100 grams of amylose, **characterized in that** said carrier comprises:
from 30% to 90% of cross-linked amylose, and
from 10% to 30% of hydroxypropylmethylcellulose (HPMC) with a viscosity higher than or equal to 4000 mPa.s,
said percentages being expressed by weight with respect to the total weight of the tablet.

2. Tablet according to Claim 1, **characterized in that** the carrier also contains at least one additional ingredient selected from the group consisting of pharmaceutically acceptable filling agents, flow agents, granulation agents, mould release agents and disintegrants.

3. Tablet according to Claim 2, **characterized in that** the active ingredient and the carrier based on cross-linked amylose are in the form of powders which are mixed and compressed to obtain the desired tablet, and **in that** the cross-linked amylose used has been prepared with 1 to 6 grams of cross-linking agent per 100 grams of amylose.

4. Tablet according to any one of Claims 1 to 3, **characterized in that** said tablet is of the matrix type and **in that** the HPMC contained in the carrier has a viscosity higher than 4000 mPa.s.

5. Tablet according to Claim 4, **characterized in that** the HPMC contained in the carrier is of the HPMC 2208 type.

6. Tablet according to Claim 5, **characterized in that** the HPMC contained in the carrier is of the HPMC 2208 type and has a viscosity equal to 100000 mPa.s.

7. Tablet according to Claim 6, **characterized in that** it comprises 20% by weight of HPMC 2208 with a viscosity equal to 100000 mPa.s.

8. Tablet according to Claim 7, **characterized in that** it comprises 10% by weight of active ingredient and **in that** the cross-linked amylose has been prepared with 3.5 grams of epichlorohydrin used as cross-linking agent per 100 grams of amylose.

9. Tablet according to Claim 4, **characterized in that** the HPMC contained in the carrier is of the HPMC 2910 type.

10. Tablet according to any one of Claims 1 to 3, **characterized in that** said tablet is of the double-core type.

11. Tablet according to Claim 10, **characterized in that** said double-core tablet includes a core containing a given amount of active ingredient and an external coating containing another amount of the same active ingredient or of another active ingredient mixed and compressed with said carrier containing cross-linked amylose and HPMC.

12. Tablet according to Claim 11, **characterized in that** the core also includes a carrier based on cross-linked amylose.

13. Tablet according to Claim 11 or 12, **characterized in that** the HPMC contained in the carrier or carriers is selected from the group consisting of those of the HPMC 2208 and HPMC 2910 type.

14. Use of HPMC according to Claim 1, with a viscosity higher than or equal to 4000 mPa.s, in a medicament consisting of a controlled release pharmaceutical tablet for oral administration comprising up to 60% by weight of active ingredient.

15. Use according to Claim 14 as adjuvant for controlling the effect of enzymes on a carrier based on amylose cross-linked with the aid of a cross-linking agent in quantities corresponding to 0.1 gram of cross-linking agent per 100 grams of amylose.
